# EUROPEAN PATENT SPECIFICATION

(11) **EP 2 093 194 B1**
(45) Date of publication and mention of the grant of the patent: **21.03.2012**
(21) Application number: 07832814.3
(22) Date of filing: 29.11.2007
(51) Int. Cl.: C09K 11/54, C09C 1/02, C01G 9/02, C09K 11/08, C12Q 1/00, G01N 21/64, G01N 21/78, G01N 33/543, G01N 33/574, G01N 33/58, C09C 1/04

(54) **FLUORESCENT LABELING AGENT AND FLUORESCENT LABELING METHOD**
FLUORESZENZMARKIERUNGSMITTEL UND FLUORESZENZMARKIERUNGSVERFAHREN
AGENT DE MARQUAGE FLUORESCENT ET PROCÉDÉ DE MARQUAGE FLUORESCENT

(30) Priority: 01.12.2006 JP 2006326347
(43) Date of publication of application: 26.08.2009
(73) Proprietor: National University Corporation Shimane University, Matsue-shi, Shimane 690-8504 (JP)
(72) Inventor: FUJITA, Yasuhisa, Matsue-shi Shimane 690-8504 (JP); NAKAMURA, Morihiko, Izumo-shi Shimane 693-8501 (JP)
(74) Representative: HOFFMANN EITLE
(86) International application number: PCT/JP2007/073114
(87) International publication number: WO 2008/066138

(56) References cited:
- WO-A1-2005/123874
- WO-A2-2005/060610
- JP-A- 2002 515 889
- JP-A- 2003 028 797
- JP-A- 2003 524 147
- JP-A- 2005 060 145
- JP-A- 2006 070 250
- JP-A- 2007 023 058
- US-A1- 2005 161 662
- US-A1- 2006 263 908
- US-B1- 6 548 168
- WU Y L ET AL: "Surface modifications of ZnO quantum dots for bio-imaging" NANOTECHNOLOGY, IOP, BRISTOL, GB, vol. 18, no. 21, 30 May 2007 (2007-05-30), page 215604, XP020119192 ISSN: 0957-4484
- SENTHILKUMAR K ET AL: "Preparation of ZnO nanoparticles for bio-imaging applications" PHYSICA STATUS SOLIDI. B, BASIC RESEARCH, AKADEMIE VERLAG, BERLIN, DE, vol. 246, no. 4, 1 April 2009 (2009-04-01) , pages 885-88, XP008118640 ISSN: 0370-1972 [retrieved on 2009-01-15]
- BANGAL M ET AL: "Semiconductor Nanoparticles" HYPERFINE INTERACTIONS, KLUWER ACADEMIC PUBLISHERS, DO, vol. 160, no. 1-4, 8 November 2005 (2005-11-08), pages 81-94, XP019397234 ISSN: 1572-9540
- Pierce: "Crosslinking reagents" March 2006 (2006-03), XP007911669 Retrieved from the Internet: URL:http://www.piercenet.com/files/1601361 crosslink.pdf> [retrieved on 2010-02-11]
- DAI N. ET AL.: 'CdSe/ZnS core/shell quantum dots for bio-application' PROCEEDINGS OF SPIE, ICO20: MATERIALS AND NANOSTRUCTURES vol. 6029, 21 August 2005, pages 602904-1 - 602904-6, XP008109941
- DORFMAN A. ET AL.: 'Highly sensitive biomolecular fluorescence detection using nanoscale ZnO platforms' LANGMUIR vol. 22, no. 11, 2006, pages 4890 - 4895, XP008109939
- DUFOUR E.K. ET AL.: 'Clastogenicity, photo-clastogenicity or pseudo-photo-clastogenicity: Genotoxic effects of zinc oxide in the dark, in pre-irradiated or simultaneously irradiated Chinese hamster ovary cells' MUTATION RESEARCH vol. 607, no. 2, 05 September 2006, pages 215 - 224, XP025175739
- J. Ma ET AL: "Formation and luminescence of ZnO nanoparticles embedded in MgO films", Physical Review B, vol. 71, no. 12, 1 March 2005 (2005-03-01) , XP55005594, ISSN: 1098-0121, DOI: 10.1103/PhysRevB.71.125430

## Description

### TECHNICAL FIELD

The present invention relates to a fluorescent labeling agent and a fluorescent labeling method, in particular to a fluorescent labeling agent and a fluorescent labeling method where a material not toxic to biomaterials is used.

### BACKGROUND ART

Known is a technique of labeling biomaterials with an organic fluorescent dye followed by irradiation with excitation light for fluorescence detection to thereby visualize specific target cells such as tumor cells alone. However, in general, an organic fluorescent dye degrades when activated through photoexcitation, and loses fluorescent, characteristics. Accordingly, owing to the fading property thereof, an organic fluorescent dye is problematic in that it is unsuitable for long-term measurement and for investigation of repetitive reproducibility. On the other hand, an antifading agent may be used for reducing the fading property but an antifading agent is generally toxic and may cause another problem in that it is unsuitable for use *in vivo*.

In addition, an organic fluorescent dye has other drawbacks in that its absorption transition is saturated when the intensity of the excitation light applied thereto is increased and the fluorescent intensity from the dye could not be over a predetermined level, that the dye must be excited at a specific wavelength, and that the wavelength of the excitation light is near to the wavelength of the fluorescent light and the excitation light and the fluorescent light are difficult to separate from each other.

Recently, a fluorescent labeling agent that comprises CdSe quantum dots has been studied as a substitute for an organic fluorescent dye. Quantum dots have various advantages in that they can fluorescently label target cells in a desired color by controlling the particle size thereof, that they are faded little by photoexcitation, that their energy state is quantumized and their light absorption is saturated little, and therefore they give intense fluorescent light having a sharp spectrum, and that they do not require any specific wavelength for excitation so far as the wavelength is shorter than the wavelength of the fluorescent light, and the excitation light and the fluorescent light are easy to separate from each other. In addition, quantum dots can readily label biomaterial cells, and are specifically noted as being hopeful for biological examinations and other diagnoses. In US 6,548,168 B1, a method for determining the presence of an analyte in a sample by using a coated particle-ligand composition is claimed. In Example 8 of the document SiO₂-coated ZnO nanoparticles are prepared.

Patent Reference 1: JP-A 2005-283254
Patent Reference 2: JP-A 2006-137682
Patent Reference 3: JP-A 2005-60145
Patent Reference 4: JP-A 2007-23058
Patent Reference 5: JD-A 2004-292282
Patent Reference 6: JP-T 2003-524147

### DISCLOSURE OF THE INVENTION

### PROBLEMS THAT THE INVENTION IS TO SOLVE

However, the prior-art techniques have the following problems.
At present, quantum dots under study for biological use are of CdSe, which, however, is problematic for *in vivo* application since the constitutive element, cadmium (Cd) is toxic.

Patent Reference 4 discloses a technique of using zinc oxide, which, however, is problematic in that it is for broad and low-brightness green emission based on defect emission and therefore the detection efficiency is poor and, as a result, it is far inferior to quantum dots as a labeling agent.

The present invention has been made in consideration of the above, and its object is to provide a fluorescent labeling agent and a fluorescent labeling method, in which a material not toxic to biomaterials is used, an intense fluorescent light having a sharp spectrum can be obtained, not faded by excitation light, and biomaterial cells are easy to label.

### MEANS FOR SOLVING THE PROBLEMS

To attain the above-mentioned object, the invention of claim 11 is the use of zinc oxide nanoparticles as an exciton emission source *in vivo*.

Specifically, the invention of claim 11 is the use of nontoxic zinc oxide, which makes it possible to fluorescently label cells and others with the exciton light thereof (wavelength, about 380 nm). A zinc oxide nanoparticle may be referred to as a nanocrystal of zinc oxide. When poorly crystalline, zinc oxide produces weak exciton emission; and when poorly crystalline, it is unstable in water and the crystal of itself dissolved therein. Accordingly, the zinc oxide nanoparticles in the invention are highly crystalline. Crystals with any other minor element existing therein are also within the scope of the zinc oxide nanoparticule as referred to herein so far as they may produce light emission of essentially zinc oxide exciton. For example, crystals with minor Mg existing therein are within that scope.

The invention of claim 9 is a use of zinc oxide nanoparticles as an exciton emission source *in vivo* or in vitro, wherein the zinc oxide nanoparticle is bound to a target and the exciton emission of the zinc oxide nanoparticles is utilized to make the target part shine. The invention of claim 6 is a corresponding fluorescent labelling method.

Specifically, the invention of claims 6 and 9 uses zinc oxide nanoparticles stable in water and indecomposable, which makes it possible to fluorescently label cells and others with the exciton light thereof (wavelength, about 380 nm).

The invention of claim 1 is a fluorescent labeling agent comprising zinc oxide nanoparticles and a substance capable of selectively binding to a target *in vivo* or in vitro, wherein the substance is bound to the zinc oxide nanoparticles via at least one binder, and wherein the surface of the zinc oxide nanoparticles is coated with a coating film of MgₓZn₁₋ₓO, with x being defined as 0 < x ≤ 1.

A Specifically, the invention of claim 1 makes it possible to fluorescently label a specific target of a biomaterial with the exciton light. At least one binder may be a substance directly binding to both the zinc oxide nanoparticles and the substance capable of selectively binding to a target, meaning that the two may be bound together via plural types of binders. Binding via plural types of binders includes an embodiment where a binder A binds to a zinc oxide nanoparticle at one end thereof and binds, at the other end thereof, to another binder B binding to a substance capable of selectively binding to a target at one end thereof, in which the binder A binds to the binder B at the other end thereof. In addition, still another binder C binding the binder A and the binder B may exist therebetween. The target *in vivo* or *in vitro* includes not only normal cells but also tumor cells and others not normal cells, and are meant to indicate the cells and other biological substances that are targeted by the person who carries out fluorescent labeling thereon. Those prepared by pretreating such cells, biological substances and others so that a fluorescent labeling agent may selectively bind to them are also within the scope of the target in the invention.

Specifically, in the invention of claim 1, the surface of zinc oxide is coated with a material having a larger band gap than that of zinc oxide, whereby the electron and the hole necessary for light emission are sealed up inside the zinc oxide nanoparticles and, as a result, a specific target in a biomaterial can be effectively or efficiently fluorescently labeled.

At least one binder may be a substance directly binding to both the coating film and the substance capable of selectively binding to a target, meaning that the two may be bound together via plural types of binders. Binding via plural types of binders includes an embodiment where a binder A binds to the coating film at one end thereof and binds, at the other end thereof, to another binder B binding to a substance capable of selectively binding to a target at one end thereof, in which the binder A binds to the binder B at the other end thereof. In addition, still another binder C binding the binder A and the binder B may exist therebetween.

Binder A and binder B, if any, in claim 1 preferably bind to each other via an amide bond through dehydrating condensation from the viewpoint of the stability thereof.

The invention of claim 2 is a fluorescent labeling agent subsidiary to claim 1, wherein the binder is a substance that modifies an amino group or a carboxyl group as a functional group on the particle side.

Specifically, the invention of claim 2 makes it possible to provide a water-soluble fluorescent labeling agent which does not aggregate but has good dispersibility. As the binder, there may be mentioned a carboxylic acid (including polycarboxylic acid).

The invention of claim 3 is a fluorescent label ing agent subsidiary to claim 1, wherein the binder is AEDP (3-[(2-aminoethyl)dithio]propionic acid), ASBA (4-(p-azidosalicylamido)butylamine), EDC (1-ethyl-3-[3-dimethylaminopropyl]carbodiimide hydrochloride), or sulfo-NHS (N-hydroxysulfosuccinimide)-added EDC, which binds to the substance capable of selectively binding to a target.

Specifically, the invention of claim 3 favorably binds to the substance capable of selectively binding to a target, and enhances the bindability to the particle. Sulfo-NHS is added as a stabilizer. The binder in claim 2 and the binder in claim 3 are preferably in a combination to form an amide bond as mentioned in the above.

The invention of claim 4 is a fluorescent labeling agent subsidiary to any one of claims 1 to 3, wherein the substance capable of selectively binding to a target is an antibody, an enzyme, a lectin or a nucleic acid.

Specifically, the invention of claim 4 makes it possible to easily recognize the targeted disease or the like in biological tests, etc. Naturally, nucleic acid includes DNA and RNA. Apart from the above, the substance capable of selectively binding to a target may further include biologically-active substances (e.g., hormone, cytokine, growth factor), receptors, glucides (carbohydrates), lipids, etc.

The invention of claim 5 is a fluorescent labeling agent subsidiary to any one of claims 1 to 4, wherein the target is a tumor cell, a leukemia cell, a virus-infected cell, or a normal cell, a protein, an enzyme or a nucleic acid.

Specifically, the invention of claim 5 makes it possible to recognize tumor and others in a simplified manner. The tumor cell shall include both benign and malignant cells. The invention of claim 5 further makes it possible to recognize various biological substances not only on the surfaces of cells but also inside cytoplasms in a simplified manner.

The invention of claim 10 is a fluorescent labeling method, wherein the exciton emission of zinc oxide nanoparticles is utilized *in vivo*.

Specifically, the invention of claim 10 uses nontoxic zinc oxide and makes it possible to fluorescently label cells and others with the exciton light thereof (wavelength, about 380 nm).

The invention of claim 7 is a fluorescent labeling method, wherein a substance capable of selectively binding to a target *in vivo* or *in vitro* is bound to a zinc oxide nanoparticle via at least one binder, and this is bound to a target, and then irradiated with UV rays as excitation light to thereby make the target part shine by the exciton emission of zinc oxide.

Specifically, the invention of claim 7 makes it possible to fluorescently label a specific target of a biomaterial with the exciton light. The excitation light may have any wavelength shorter than the wavelength of the exciton light (about 380 nm) and falling within a range not having any specific influence on biomaterials. For it, for example, there may be mentioned a pulse laser with consecutive oscillations such as helium-cadmium laser (325 nm) and nitrogen laser (wavelength, 337 nm), and UV lamp light sources such as mercury lamp, etc.

The invention of claim 8 is a fluorescent labeling method subsidiary to claim 7, wherein the surface of zinc oxide nanoparticles is coated with a coating film selected from ZnS, MgₓZn₁₋ₓO (where 0 < x ≤ 1), SiO₂, and combination thereof.

Specifically, in the invention of claim 8, the surface of zinc oxide is coated with a material having a larger band gap than that of zinc oxide, whereby the electron and the hole necessary for light emission are sealed up inside the zinc oxide nanoparticles and, as a result, a specific target in a biomaterial can be effectively or efficiently fluorescently labeled. The nanoparticles may be coated with a plurality of ZnS, MgₓZn₁₋ₓO and SiO₂, as combined.

Needless-to-say, the techniques for claim 2 to claim 5 are applicable also to the fluorescent labeling method of claims 7, 8 and 10.

### EFFECT OF THE INVENTION

In the invention, used is zinc, an indispensable element in living bodies, and as the compound thereof, used is nontoxic zinc oxide. Accordingly, the invention makes it possible to provide a fluorescent labeling agent and a fluorescent labeling method that are highly safe and are utilizable in *vivo* or *in vitro.* Further in the invention, the exciton light of zinc oxide is utilized, and therefore intense emission with little fading can be obtained like that from quantum dots. Accordingly, the invention has another advantage in that it simplifies fluorescent assay. Moreover, the surface of the zinc oxide nanoparticle is coated with a substance having a large band gap, ad the exciton emission is thereby stabilized. In addition, the fluorescent labeling agent of the invention is such that the zinc oxide nanoparticle therein is bound to a target via a binder, and therefore, it facilitates fluorescent labeling of various biomaterials.

### BEST MODE FOR CARRYING OUT THE INVENTION

It is known that, in a bulk "crystal" of zinc oxide of high crystallinity, the exciton where the electron and the hole are in a mutually restricted state can exist as such, not dissociating even at room temperature. When zinc oxide in that state is irradiated with light having a wavelength not longer than 380 nm, then it absorbs the light to form an exciton, and gives exciton-derived high-efficiency light emission having a peak wavelength of about 380 nm. The energy level of the exciton concentrates to a specific energy like that of a hydrogen atom composed of a proton and an electron, and therefore, the exciton emission gives an intense light having a sharp spectrum like that from a quantum dot.

On the other hand, for a long time, "fine particles" of zinc oxide are produced through vaporization of zinc in an oxygen atmosphere, and they are a nontoxic material used for baby powder, medicines, cosmetics and others in the field relating to human bodies. However, the fine particles of zinc oxide produced according to the above method generally have poor crystallinity. Therefore, they have some drawbacks in that (1) the green-defective emission is strong and the exciton emission is weak; (2) the agglomeration is strong to form white powdery blocks, which are difficult to disperse in water; and (3) they are unstable in water and the crystals themselves dissolve therein.

Specifically, zinc oxide is known in some degree as a fluorescent substance that gives broad green defect emission; however, the exciton emission thereof is a study subject exclusively limited to the field of condensed matter physics. Naturally, therefore, there should not be an idea of using zinc oxide as a light source material for exciton emission in the field of health occupation that differs from the above in point of the directionality, and besides, no one could hit on an idea of using zinc oxide as a fluorescent labeling agent in *vivo* or *in vitro.*

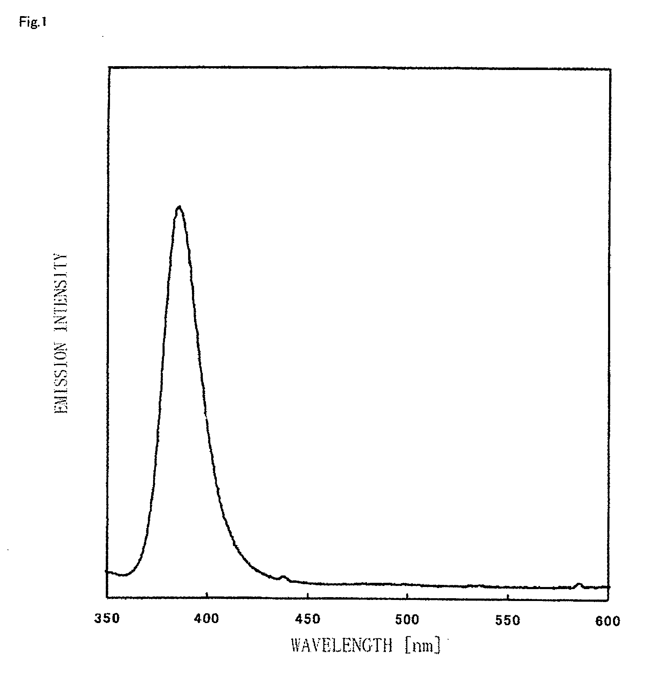

On the other hand, the present inventors have succeeded in producing zinc oxide nanoparticles of excellent crystallinity that are essentially for exciton emission, according to a gas evaporation method of vaporizing zinc in an oxygen-containing gas through arc discharging therein (see Patent Reference 3). In fact, when the zinc oxide nanoparticles produced according to the method were excited with a He-Cd laser having a wavelength of 325 nm at room temperature and analyzed with a spectrometer, then an exciton light having a peak at about 380 nm given by them was observed in a simplified manner, as shown in Fig. 1. The half-value width was at most about 25 nm, and the exciton light of this material gave a sharp spectrum.

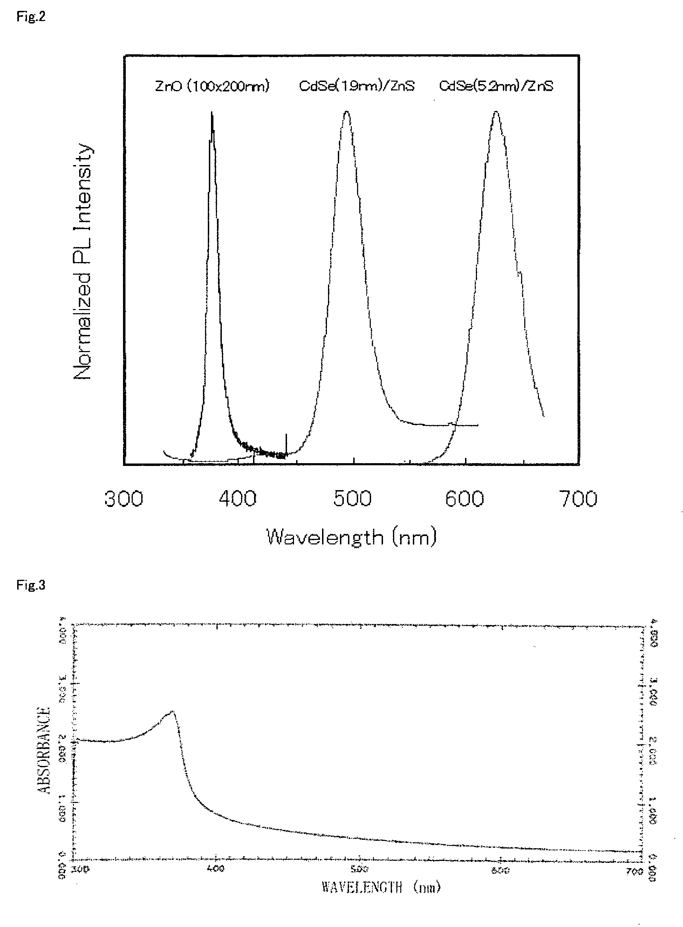

The fluorescent spectrum of the exciton light of the obtained zinc oxide nanoparticles was compared with the fluorescent spectrum of Evident's ZnS-coated CdSe quantum dots, as shown in Fig. 2. The two could not be compared with each other in point of the emission intensity since the dispersion concentration and the detection sensitivity differ depending on the wavelength; however, as shown in the drawing, it was confirmed that the present zinc oxide nanoparticles of good crystallinity gave sharp fluorescence comparable to or more than that of the quantum dots.

In general, the intensity of the exciton light is characterized by increasing in accordance with the intensity of the incident light, and differs from that in the emission by fluorescent dye of which the intensity is saturated; and the availability of the present zinc oxide nanoparticles is great. Further, quantum dots require regulation or control of their particle size to a nanosize level; however, the exciton light of the zinc oxide nanoparticles produced by the present inventors could have a sharp spectrum of high brightness comparable to that of quantum dots even though their particle size is neither regulated nor controlled, and therefore it may be said that their production efficiency is excellent.

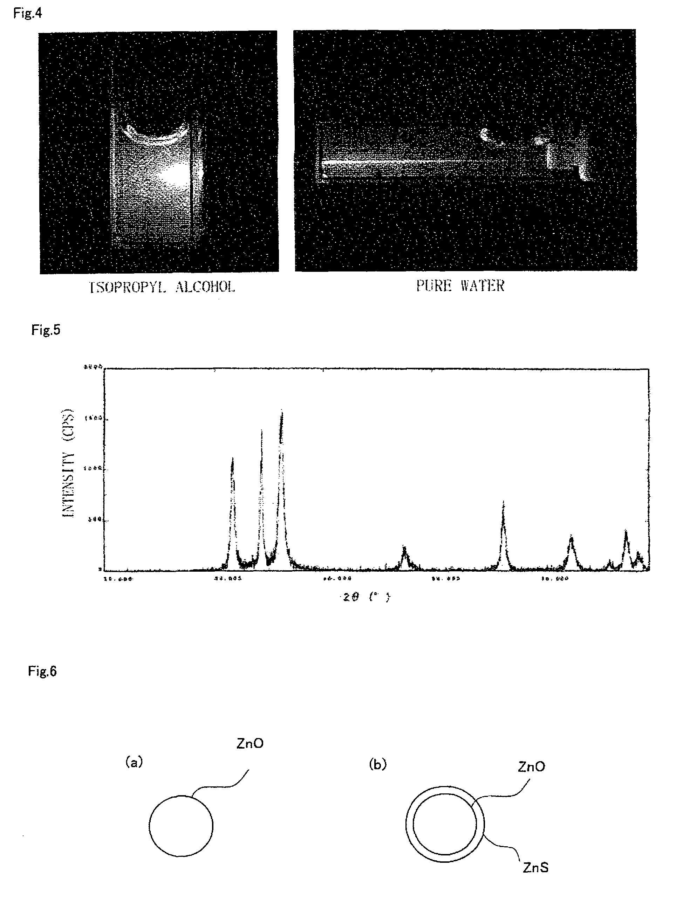

In addition to the above-mentioned findings, the present inventors have further confirmed that the obtained zinc oxide nanoparticles of good crystallinity are stable in water (room temperature) and the crystals themselves can disperse therein still as nanoparticles. Fig. 3 shows the data of an absorption spectrum of the obtained zinc oxide nanoparticles dispersed in water, analyzed with a spectrophotometer. The exciton absorption is seen at about 375 nm, which indicates good dispersion of the nanoparticles. The present inventors have further confirmed that the obtained zinc oxide nanoparticles gave exciton emission also in isopropyl alcohol and in pure water, not aggregated but stably dispersed therein, as shown in Fig. 4 (the light source is He-Cd laser having a wavelength of 325 nm).

Based on these findings, the present inventors have completed the invention.
The method for producing zinc oxide nanoparticles is not specifically defined; however, according to the method of Patent Reference 3, particles of good crystallinity can be produced inexpensively, of which the particle size falls within a range of approximately from 50 nm to 200 nm and the mean particle size is approximately 100 nm or so.

Crystals having a smaller size can be produced, for example, by mixing melted zinc acetate and sodium hydroxide in ethanol followed by stirring at room temperature for 90 minutes. Fig. 5 shows an X-ray diffractiometry pattern of the zinc oxide nanoparticles produced according to the method followed by centrifugation and drying. The peaks in this drawing confirm the formation of zinc oxide crystal. The expanse of the diffraction peaks indicates that the mean particle size may be about 30 nm. Apart from this, zinc oxide quantum dots having a size of about a few nm or so may be produced according to a sol-gel method using zinc acetate, anhydrous ethanol and lithium hydroxide. In the invention, the particle size is not specifically defined, and may be selected in accordance with the use thereof. For example, when introduction into blood vessels is taken into consideration, the particle size is preferably at most dozens of nm or so.

When the surfaces of zinc oxide nanoparticles are coated with ZnS, MgₓZn₁₋ₓO (where 0 < x ≤ 1) or SiO₂, then the carriers excited by irradiation with light can be efficiently prevented from scattering away, and therefore the nanoparticles may secure stable fluorescent emission with no trouble of extinction by the neighboring substances. The zinc oxide nanoparticle coated with the above compound is hereinafter suitably referred to as a shell-coated zinc oxide nanoparticle. Fig. 6 shows graphic views of (a) zinc oxide nanoparticle, and (b) shell-coated zinc oxide nanoparticle.

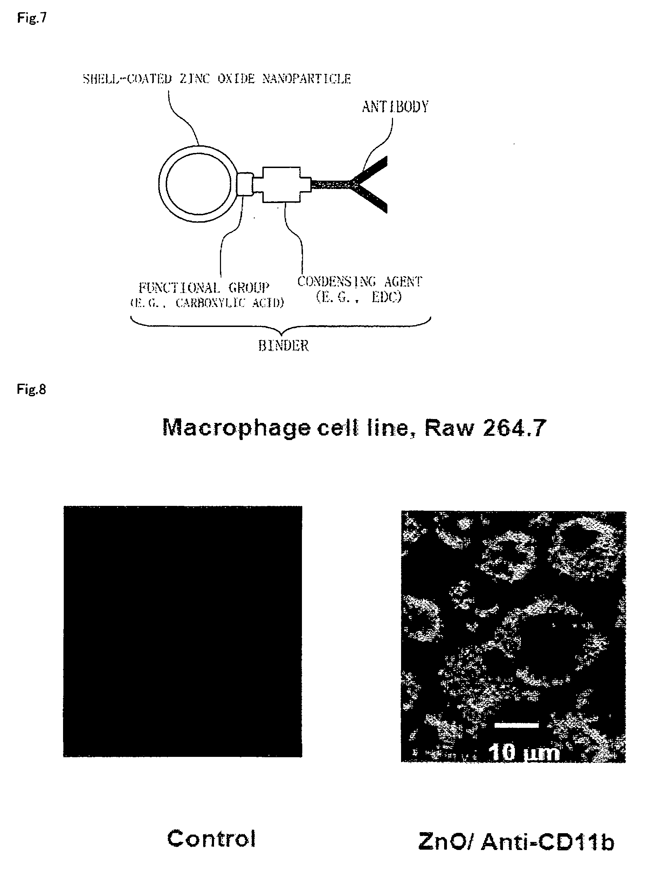

A zinc oxide nanoparticle or a shell-coated zinc oxide nanoparticle may be bound to an antibody, an enzyme, a lectin or a nucleic acid (hereinafter this may be suitably referred to as an antibody or the like) via.a binder (see Fig. 7). In this case, the zinc oxide nanoparticle or the shell-coated zinc oxide nanoparticle is preferably surface-treated with a carboxylic acid (including a polycarboxylic acid). This may enhance the solubility or the dispersibility of the particle and may enhance the bindability thereof with an antibody or the like.

For the surface treatment, first, the nanoparticle is reacted with a carboxylic acid, for example, in the presence of a halogenohydrocarbon (e.g., chloroform, methylene chloride), an alcohol (e.g., methanol, ethanol), an ester (e.g., acetate, phthalate), or any other organic solvent (e.g., acetic acid, acetonitrile, dimethylsulfoxide), at room temperature for 2 hours or so. Next, the solvent is evaporated away, and the unreacted reagents are removed through ultrafiltration. Accordingly, a water-soluble zinc oxide nanoparticle can be obtained. Preferred examples of the carboxylic acid include mercaptosuccinic acid, 2,3-dimercaptosuccinic acid, DL-cysteine, etc. In addition, the nanoparticle may be modified with an amino group.

On the other hand, the antibody or the like is bound to a condensing agent. The antibody or the like is bound to the side of the particle through dehydrating condensation with the condensing agent. Examples of the condensing agent include AEDP, ASBA, or EDC. In particular, EDC is preferred as having practical results. In case where EDC is used, Sulfo-NHS is preferably added thereto as an auxiliary agent.

As the binder, preferred is a combination of EDC and mercaptosuccinic acid, EDC and 2,3-dimercaptosuccinic acid, or EDC and DL-cysteine. Sulfo-NHS may be optionally used as an auxiliary agent.

Specifically, in one embodiment, a fluorescent labeling agent can be obtained by reacting a zinc oxide nanoparticle or a shell-coated zinc oxide nanoparticle with a carboxylic acid in an alcohol, an ester, an ether or any other solvent, then evaporating away the solvent, and finally reacting the carboxyl group on the particle side and the amino group on the side of the antibody or the like through dehydrating condensation with a condensing agent for use in peptide synthesis. In this, the antibody or the like is previously bound to the condensing agent.

In another embodiment, a binder previously processed for dehydrating condensation is prepared, and a zinc oxide nanoparticle and an antibody or the like may be bound thereto in order to produce a fluorescent labeling agent. For example, EDC is first reacted with mercaptosuccinic acid, using Sulfo-NHS as an auxiliary agent, and thereafter reacted with zinc oxide, and finally reacted with an antibody or the like to obtain the intended fluorescent labeling agent. It has been confirmed that this method is effective especially when shell-free zinc oxide is used.

Concrete production methods are introduced hereunder. First, according to the method of Patent Reference 3 (JP-A 3005-60145) disclosed by the present inventors, zinc oxide nanoparticles were produced. These were dispersed in a chicroform solution and the concentration was controlled to be 20 mg/ml. To 10 ml of the dispersion, added was 2 ml of acid/methanol solution (25 mg/ml, by Sigma), and reacted at room temperature for 12 hours. The reaction liquid was centrifuged (12,000 × g) at 4°C for 30 minutes, and the remaining chloroform and methanol were removed through suction with an evaporator thereby giving a dry powder of water-soluble zinc oxide nanoparticles. 5 ml of PBS (-) solution (pH 7.3) was added thereto to dissolve the dry powder. The resulting solution was centrifuged (12, 000 × g) at 4°C for 30 minutes to remove the precipitate (insoluble matter). The obtained, zinc oxide nanoparticles-containing supernatant was ultrafiltered through Microcon (molecular weight cut-off: 3000 MW, by Millipore) to remove the molecules having a molecular weight of at most 3000 including the unreacted reagents. As a result, herein obtained were zinc oxide nanoparticles of good dispersibility that had been surface-treated with a carboxylic acid.

To this solution, added was an EDC solution controlled to have a concentration of 5 mg/ml with distilled water, and reacted at room temperature for 2 hours. The unreacted zinc oxide nanoparticles and reagents and the reaction side-products were removed through ultrafiltration with Microcon (molecular weight cut-off: 3000 MW, by Millipore). Next, the obtained fluorescent labeling agent (1.5 ml) and 200 µl of an antibody solution (2 mg) controlled with a PBS solution were mixed to give the intended, water-soluble fluorescent agent. The antibody is an anti-Cd11b antibody capable of recognizing macrophage cancer cells (Raw264.7).

The fluorescent labeling agent obtained according to the above-mentioned production method was actually subjected to antibody reaction, and irradiated with a laser ray (wavelength, 325 nm). As a result, light emission from the surface of the mouse macrophage cancer cell (Raw264.7), or that is, from the antibody reaction part was confirmed. Further, it was confirmed that the fluorescent labeling agent obtained according to the production method was stable, not forming a precipitate even after stored at 4°C for 1 week.

The following are modifications of the above-mentioned production example. In place of 2 ml of mercaptosuccinic acid solution (25 mg/ml, by Sigma), usable are 2,3-dimercaptosuccinic acid solution (25 mg/ml, by Sigma) or DL-cysteine solution (25 mg/ml, by Sigma). The reaction time was 12 hours at room temperature in the above; however, it was confirmed that the fluorescent labeling agent could be produced even when the reaction time was 1 hour. It was further confirmed that the condition in the first centrifugation could be 1000 × g, 5 minutes and 4°C.

It was also confirmed that sulfo-NHS-added EDC could be used as the condensing agent, and its concentration could be controlled with distilled water and MES buffer.

A production example of another fluorescent labeling agent is described. In this example, streptoavidin is used. First, zinc oxide nanoparticles (2 mg) surface-treated with a carboxylic acid (mercaptosuccinic acid or DL-cysteine) is dissolved in 0.1 M MES buffer (1 ml). To the solution, added are EDC (0.5 mg) and sulfo-NHS (1 mg), and reacted at room temperature for 15 minutes. Next, EDC is inactivated with 1.7 µl of 2-mercaptoethanol, and then the unreacted EDC is separated through a PBS-equilibrated de-salting column (Zeta Desalting Column, by Pierce). Finally, 1 mg of streptoavidin was added to 1 ml of the zinc oxide nanoparticles-containing solution and reacted for crosslinking at room temperature for 2 hours, thereby giving the intended water-soluble fluorescent labeling agent.

In labeling and investigation, first, a commercially-available, biotin-labeled anti-Cd11b antibody (by Biolegend) is previously reacted with mouse macrophage Raw264.7. To this, added is the fluorescent labeling agent-containing solution (50 µg/ml) obtained according to the above-mentioned method, and reacted at room temperature for 2 hours. This is washed three times or so with PBS to remove the unreacted fluorescent labeling agent. After washed, this is irradiated with light, whereupon the fluorescent light by exciton emission can be detected with an epi-illumination fluorescent microscope.

The above-mentioned fluorescent labeling agents are examples of using zinc oxide nanoparticles. Fluorescent labeling agents can be produced similarly, using shell-coated zinc oxide nanoparticles. In place of the antibody, a Lectin, an enzyme or a nucleic acid may be bound to the zinc oxide nanoparticles to produce fluorescent labeling agents. In the final step of producing the fluorescent labeling agents, protein A may be used in case where an antibody is bound to the nanoparticles, or a saccharide-fixed chromatographic resin (e.g., melibiose agarose) may be used in case where a lectin is bound thereto, whereby the fluorescent labeling agents can be purified to have a high purity.

Next, concrete labeling results are shown below.

### <Labeling Result 1>

The present fluorescent labeling agent containing an anti-Cd11b antibody was reacted with target cells mouse macrophage Raw264.7 at room temperature for 2 hours, then washed three times with PBS to remove the unreacted antibody, and then checked for fluorescent emission with an epi-illumination fluorescent microscope. In the observation, used were filters of exciter 340, beam splitter 365 and emitter 380. Fig. 8 shows photographs of checkup results. The left is control, and the right is the labeling result. As shown, fluorescent light (λmax = about 380 nm) based on the zinc oxide nanoparticles was obviously detected on the surfaces of the target cell membranes,

In preparing the fluorescent labeling agent used herein, mercaptosuccinic acid was used as one material of the binder and reacted at room temperature for 12 hours. The condition of the first centrifugation to remove the precipitate was 12000 × g, 30 minutes and 4°C. As another material of the binder, sulfo-NHS-added EDC was used.

### <Labeling Result 2>

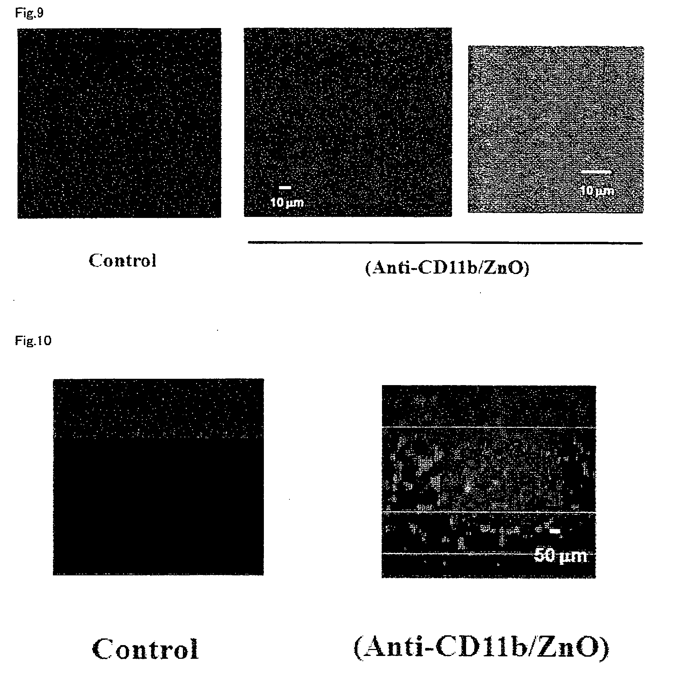

In the same manner as that for the labeling result 1 and using filters of exciter 335, beam splitter 365 and emitter 380, the fluorescent emission was detected. Fig. 9 shows photographs of checkup results. The left is control, the center is the labeling result, and the right is an enlarged view. As shown, fluorescent emission on the surfaces of the target cells is obvious.

In preparing the fluorescent labeling agent used herein, mercaptosuccinic acid was used as one material of the binder and reacted at room temperature for 1 hour. The condition of the first centrifugation to remove the precipitate was 1000 × g, 5 minutes and 4°C; and the condition of the second centrifugation to remove the supernatant is 12000 × g, 20 minutes and 4°C. As another material of the binder, sulfo-NHS-added EDC was used.

### <Labeling Result 3>

A lyzate (cell extract) of mouse macrophage Raw264.7 was dot-blotted on a methyl alcohol-processed nitrocellulose membrane, and reacted with the present fluorescent labeling agent containing an anti-Cd11b antibody, at room temperature for 2 hours. This was washed three times with PBS to remove the unreacted antibody, and checked for fluorescent emission with an epi-illumination fluorescent microscope. Fig. 10 shows photographs of checkup results. The left is control, and the right is the labeling result. As shown, the labeled target emitted light as a whole, and the dots are obvious. The shadow is caused by the influence of the paper surface on the photograph. The same fluorescent labeling agent as that used in the above labeling result 2 was used herein.

As shown in the above, use of the present fluorescent labeling agent enables fluorescent labeling detection of target cells. Further, since the present fluorescent labeling agent is not toxic, it can be used not only *in vitro* but also *in vivo*.

### INDUSTRIAL APPLICABILITY

As in the above, the zinc oxide nanoparticles to constitute the fluorescent labeling agent of the invention are a non-toxic material and are characterized in that they give strong fluorescent emission like quantum dots, that they fade little, and that they can fluorescently label a target with ease, and therefore, the nanomaterial can be used for various application. One application example of the invention is biological tests. For example, a tissue is partly collected from a patient during surgical operation, the fluorescent labeling agent is added thereto and irradiated with laser light to check for the presence of absence of light emission at around 380 nm, whereby it is possible to immediately determine as to whether the tissue is positive or negative. In addition, since the fluorescent labeling agent of the invention is a nontoxic material, it enables clinical tests for skin cancer and other tumors with an endoscope.

As combined with quantum dots, the invention enables multiple labeling. As shown in Fig. 1, since the fluorescent labeling agent of the invention has a narrow and sharp spectrum, its fluorescent emission can be clearly differentiated from any other fluorescent emission and its labeling accuracy is thereby increased.

### BRIEF DESCRIPTION OF THE DRAWINGS

[Fig. 1] It is an exciton emission spectrum of zinc oxide nanoparticles produced according to gas evaporation method.
[Fig. 2] It is a graph of comparing the fluorescent light spectrum of the exciton light of zinc oxide nanoparticles for use in the invention, with the fluorescent light spectrum of Evident's ZnS-coated CdSe quantum dots.
[Fig. 3] It is a graph showing the results of the absorption spectrum of a dispersion in water of the zinc oxide nanoparticles obtained herein, as measured with a spectrophotometer.
[Fig. 4] It is a photograph showing the light emission from zinc oxide nanoparticles obtained herein. The left is a dispersion of the nanoparticles in isopropyl alcohol; and the right is a dispersion thereof in pure water.
[Fig. 5] It is an X-ray diffraction pattern of zinc oxide nanoparticles having a mean particle size of 30 nm.
[Fig. 6] It shows graphic views of (a) zinc oxide nanoparticle, and (b) shell-coated zinc oxide nanoparticle.
[Fig. 7] It shows one constitution example of the fluorescent labeling agent of the invention, and is a graphic view showing a mode of binding a shell-coated zinc oxide nanoparticle, as surface-modified with a functional group, to an antibody via a binder.
[Fig. 8] It shows one example of a labeling result with target cells.
[Fig. 9] It shows one example of a labeling result with target cells.
[Fig. 10] It shows microscopic pictures of a labeling result with a lyzate.

## Claims

1. A fluorescent labeling agent comprising zinc oxide nanoparticles and a substance capable of selectively binding to a target *in vivo* or *in vitro*,
wherein the substance is bound to the zinc oxide nanoparticles via at least one binder, and
wherein the surface of the zinc oxide nanoparticles is coated with a coating film of MgₓZn₁₋ₓO, with x being defined as 0 < x ≤ 1.

2. The fluorescent labeling agent according to Claim 1, wherein the binder is a substance that modifies an amino group or a carboxyl group as a functional group on the particle side.

3. The fluorescent labeling agent according to Claim 1, wherein the binder is AEDP (3-[(2-aminoethyl)dithio]propionic acid), ASBA (4-(p-azidosalicylamido)butylamine), EDC (1-ethyl-3-[3-dimethylaminopropyl]carbodiimide hydrochloride), or sulfo-NHS (N-hydroxysulfosuccinimide)-added EDC.

4. The fluorescent labeling agent according to any one of Claims 1 to 3, wherein the substance capable of selectively binding to a target is an antibody, an enzyme, a lectin or a nucleic acid.

5. The fluorescent labeling agent according to any one of Claims 1 to 4, wherein the target is a tumour cell, a leukaemia cell, a virus-infected cell, or a normal cell, a protein, an enzyme or a nucleic acid.

6. A fluorescent labeling method, wherein a zinc oxide nanoparticle is bound to a target and the exciton emission of zinc oxide nanoparticles is utilized *in vivo* or *in vitro* to make the target part shine.

7. A fluorescent labeling method, wherein a substance capable of selectively binding to a target *in vivo* or *in vitro* is bound to a zinc oxide nanoparticle via at least one binder, and this is bound to a target, and then irradiated with UV rays as excitation light to thereby make the target part shine by the exciton emission of zinc oxide.

8. The fluorescent labeling method according to Claim 7, wherein the surface of zinc oxide nanoparticles is coated with a coating film selected from ZnS, MgₓZn₁₋ₓO with x being defined as 0 < x ≤ 1, SiO₂, and combinations thereof.

9. Use of zinc oxide nanoparticles as an exciton emission source *in vivo* or *in vitro*, wherein a zinc oxide nanoparticle is bound to a target and the exciton emission of zinc oxide nanoparticles is utilized to make the target part shine.

10. A fluorescent labeling method, wherein the exciton emission of zinc oxide nanoparticles is utilized in *vivo.*

11. Use of zinc oxide nanoparticles as an exciton emission source in *vivo.*

## Patentansprüche

1. Fluoreszenz-Markierungsmittel, welches Zinkoxid-Nanopartikel und eine Substanz, die in der Lage ist, selektiv an ein Target in vivo oder in vitro zu binden, umfasst,
wobei die Substanz an die Zinkoxid-Nanopartikel durch mindestens ein Bindemittel gebunden ist, und
wobei die Oberfläche der Zinkoxid-Nanopartikel mit einem Beschichtungsfilm aus MgₓZn₁₋ₓO beschichtet ist, in welchem x als 0 < x ≤ 1 definiert ist.

2. Fluoreszenz-Markierungsmittel gemäß Anspruch 1, wobei das Bindemittel eine Substanz ist, die eine Aminogruppe oder eine Carboxylgruppe als funktionelle Gruppe auf der Partikelseite modifiziert.

3. Fluoreszenz-Markierungsmittel gemäß Anspruch 1, wobei das Bindemittel AEDP (3-[(2-Aminoethyl)dithio]-propionsäure), ASBA (4-(p-Azidosalicylamido)butylamin), EDC (1-Ethyl-3-[3-dimethylaminopropyl]carbodiimidhydrochlorid) oder Sulfo-NHS (N-Hydroxysulfosuccinimid)-addiertes EDC ist.

4. Fluoreszenz-Markierungsmittel gemäß einem der Ansprüche 1 bis 3, wobei die Substanz, die in der Lage ist, selektiv an ein Target zu binden, ein Antikörper, ein Enzym, ein Lectin oder eine Nucleinsäure ist.

5. Fluoreszenz-Markierungsmittel gemäß einem der Ansprüche 1 bis 4, wobei das Target eine Tumorzelle, eine Leukämiezelle, eine virusinfizierte Zelle oder eine normale Zelle, ein Protein, ein Enzym oder eine Nucleinsäure ist.

6. Fluoreszenz-Markierungsverfahren, worin ein Zinkoxid-Nanopartikel an ein Target gebunden wird und die Exzitonemission der Zinkoxid-Nanopartikel in vivo oder in vitro genutzt wird, um das Targetteil zum Strahlen zu bringen.

7. Fluoreszenz-Markierungsverfahren, worin eine Substanz, die in der Lage ist, selektiv in vivo oder in vitro an ein Target zu binden, an einen Zinkoxid-Nanopartikel mit mindestens einem Bindemittel gebunden wird, und dieses an ein Target gebunden wird und dann mit UV-Strahlen als Anregungslicht bestrahlt wird, um **dadurch** das Targetteil durch die Exzitonemission des Zinkoxids zum Strahlen zu bringen.

8. Fluoreszenz-Markierungsverfahren gemäß Anspruch 7, worin die Oberfläche der Zinkoxid-Nanopartikel mit einem Beschichtungsfilm ausgewählt aus ZnS, MgₓZn₁₋ₓO, in dem x als 0 < x ≤ 1 definiert ist, SiO₂ und Kombinationen daraus beschichtet ist.

9. Verwendung von Zinkoxid-Nanopartikeln als Exzitonemissionsquelle in vivo oder in vitro, wobei ein Zinkoxid-Nanopartikel an ein Target gebunden wird, und die Exzitonemission der Zinkoxid-Nanopartikel verwendet wird, um das Targetteil zum Strahlen zu bringen.

10. Fluoreszenz-Markierungsverfahren, worin die Exzitonemission der Zinkoxid-Nanopartikel in vivo verwendet wird.

11. Verwendung von Zinkoxid-Nanopartikeln in vivo als Exzitonemissionsquelle.

## Revendications

1. Agent de marquage fluorescent comprenant des nanoparticules d'oxyde de zinc et une substance apte à se lier sélectivement à une cible *in vivo* ou *in vitro,*
dans lequel la substance est liée aux nanoparticules d'oxyde de zinc par l'intermédiaire d'au moins un liant, et
dans lequel la surface des nanoparticules d'oxyde de zinc est revêtue avec un film de revêtement de MgₓZn₁₋ₓO, avec x étant défini comme 0 < x ≤ 1.

2. Agent de marquage fluorescent selon la revendication 1, dans lequel le liant est une substance qui modifie un groupe amino ou un groupe carboxyle comme un groupe fonctionnel sur le côté de particule.

3. Agent de marquage fluorescent selon la revendication 1, dans lequel le liant est l'acide AEDP (3-[(2-aminoéthyl)dithio]propionique), l'ASBA (4-(p-azidosalicylamido)butylamine), le chlorhydrate d'EDC (1-éthyl-3-[3-diméthylaminopropyl]carbodiimide, ou l'EDC avec ajout de sulfo-NHS (N-hydroxysulfosuccinimide).

4. Agent de marquage fluorescent selon l'une quelconque des revendications 1 à 3, dans lequel la substance apte à se lier sélectivement à une cible est un anticorps, une enzyme, une lectine ou un acide nucléique.

5. Agent de marquage fluorescent selon l'une quelconque des revendications 1 à 4, dans lequel la cible est une cellule tumorale, une cellule leucémique, une cellule infectée par un virus, ou une cellule normale, une protéine, une enzyme ou un acide nucléique.

6. Procédé de marquage fluorescent, dans lequel une nanoparticule d'oxyde de zinc est liée à une cible et l'émission d'excitons de nanoparticules d'oxyde de zinc est utilisée *in vivo* ou *in vitro* pour faire briller la partie cible.

7. Procédé de marquage fluorescent, dans lequel une substance apte à se lier sélectivement à une cible *in vivo* ou *in vitro* est liée à une nanoparticule d'oxyde de zinc par l'intermédiaire d'au moins un liant, et celui-ci est lié à une cible, et ensuite éclairée avec des rayons UV comme lumière d'excitation pour ainsi faire briller la partie cible par l'émission d'excitons de l'oxyde de zinc.

8. Procédé de marquage fluorescent selon la revendication 7, dans lequel la surface des nanoparticules d'oxyde de zinc est revêtue avec un film de revêtement choisi parmi ZnS, MgₓZn₁₋ₓO, avec x étant défini comme 0 < x ≤ 1, SiO₂, et des combinaisons de ceux-ci.

9. Utilisation de nanoparticules d'oxyde de zinc comme une source d'émission d'excitons *in vivo* ou *in vitro,* dans laquelle une nanoparticule d'oxyde de zinc est liée à une cible et l'émission d'excitons de nanoparticules d'oxyde de zinc est utilisée pour faire briller la partie cible.

10. Procédé de marquage fluorescent, dans lequel l'émission d'excitons de nanoparticules d'oxyde de zinc est utilisée *in vivo*.

11. Utilisation de nanoparticules d'oxyde de zinc comme une source d'émission d'excitons *in vivo*.
